# EUROPEAN PATENT APPLICATION

(11) **EP 4 799 638 A1**
(43) Date of publication of application: **02.09.2026**
(21) Application number: 24902716.0
(22) Date of filing: 06.12.2024
(51) Int. Cl.: A61K 39/39, A61K 39/25, A61K 39/00, A61P 31/00

(54) **IMMUNOLOGIC ADJUVANT COMPOSITION, PREPARATION METHOD THEREFOR AND USE THEREOF**

(30) Priority: 15.12.2023 CN 202311732221
(71) Applicant: Grand Theravac Life Science (Nanjing) Co., Ltd., Nanjing, Jiangsu 210032 (CN)
(72) Inventor: GE, Jun, Nanjing, Jiangsu 210032 (CN); LI, Jianqiang, Nanjing, Jiangsu 210032 (CN); SUN, Lilong, Nanjing, Jiangsu 210032 (CN); ZHOU, Tong, Nanjing, Jiangsu 210032 (CN); SUN, Jiaojiao, Nanjing, Jiangsu 210032 (CN); LIU, Ke, Nanjing, Jiangsu 210032 (CN); LIANG, Xuan, Nanjing, Jiangsu 210032 (CN); GUO, Lili, Nanjing, Jiangsu 210032 (CN)
(74) Representative: Uexküll & Stolberg
(86) International application number: PCT/CN2024/137326
(87) International publication number: WO 2025/124295

(57) **Abstract**

Provided are an immunological adjuvant composition, a preparation method and use thereof. The immunological adjuvant composition comprises an immune activator and a liposome for loading the immune activator, wherein the immune activator includes a saponin and a CpG oligodeoxynucleotide. The immunological adjuvant composition is capable of eliciting an immune response against human herpesvirus and/or hepatitis B virus in a mammal and inducing a strong level of cellular immune response. By using the liposome to simultaneously load QS21 and the CpG oligodeoxynucleotide, the two components exhibit a favorable synergistic effect in promoting cellular immunity, and can induce a stronger level of cellular immune response compared to the commercially available vaccine Shingrix.

## Description

### Cross Reference to Related Application

This application claims priority to Chinese Patent Application No. 202311732221.3, filed on December 15, 2023, the entire content of which is incorporated herein by reference.

### Technical Field

The present invention belongs to the field of biopharmaceuticals. Specifically, the present invention relates to an immunological adjuvant composition for eliciting an immune response, a preparation method and use thereof.

### Background Art

Hepatitis B virus (HBV) infection is one of the serious public health problems worldwide. HBV infection is an important cause of chronic hepatitis B, liver cirrhosis, and hepatocellular carcinoma. Commonly used drugs for clinical treatment of chronic HBV infection mainly include nucleoside analogs and interferons. However, nucleosides cannot completely eliminate cccDNA in hepatocytes, and long-term use is prone to the emergence of drug-resistant mutant strains and rebound after drug withdrawal. Interferon is not suitable for asymptomatic HBV carriers, and in patients with chronic HBV infection, the HbeAg seroconversion rate is only 33% after half a year of use, with limited therapeutic effect and relatively severe side effects.

Herpes zoster is a viral skin disease caused by varicella-zoster virus (VZV). During primary infection with VZV, the virus enters regional lymph nodes via the respiratory mucosal epithelium for replication. Virus-infected lymphocytes then enter the blood circulation through the lymphatic circulation to infect peripheral blood mononuclear leukocytes, and the virus spreads to the skin via the bloodstream, clinically manifesting as varicella. Vaccine formulation is the most effective and economical means for the prevention and control of such a disease.

Liposomes are composed of phospholipid bilayers and have a structure similar to that of cell membranes. They possess both adjuvant and carrier functions and can serve as carriers for antigens that elicit hapten-specific immune responses. Encapsulation of antigens in liposomes can protect the encapsulated antigens from degradation, thereby reducing the dose and inoculation frequency of the antigens, lowering the toxicity of the encapsulated antigens, and enhancing the ability of animals to resist challenge by high doses of pathogenic microorganisms or their toxins.

Therefore, liposomes have become ideal adjuvant carriers.

Saponins are triterpene glycosides extracted from the bark of *Quillaja saponaria.* Quil-A is a partially purified aqueous extract of saponin material, and QS21 is an HPLC-purified, non-toxic fraction of Quil-A having a complex glycoester structure. This structure comprises a quillaic acid triterpene ring structure substituted with a branched trisaccharide and a linear tetrasaccharide, in which the linear tetrasaccharide is linked to an acyl chain.

Chemically, CpG oligodeoxynucleotides are deoxyoligonucleotides containing cytosine-guanine dinucleotides. They elicit an immune response similar to that induced by natural CpG through pattern recognition receptors, can bind to Toll-like receptors on cell membranes, and effectively elicit an immune response in mammals through the TLR9 signaling pathway.

Monophosphoryl lipid A (MPL) is a lipopolysaccharide derivative with relatively low toxicity and adjuvant activity. As a TLR4 agonist, it can stimulate macrophages to produce TNF, which, together with the TNF produced, promotes NK cells to produce IFN-γ, thereby selectively activating Th1 cells.

In the prior art, patent application CN101330924A by GlaxoSmithKline Biologicals SA discloses a composite formulation comprising QS21, MPL and a liposome, which is a novel herpes zoster vaccine capable of eliciting an immune response against varicella-zoster virus in mammals (hereinafter referred to as Shingrix). The vaccine uses the liposome as a delivery carrier to coordinate with QS21 and MPL for adjuvant delivery. However, it does not disclose how QS21, MPL, and the liposome interact with one another, nor how the liposome coordinates with QS21 and MPL in adjuvant delivery. Therefore, the structure of liposomes and the interaction between liposomes and adjuvants have become the focus of current research on liposomes.

In the prior art, Exicure, Inc. has conducted in-depth research on the structure of liposomes and the interaction between liposomes and adjuvants. Patent application CN106535876A by this company describes that the mode of action between liposomes and oligonucleotides involves conjugating the oligonucleotides via a linker molecule to anchor them to the hydrophobic region of the phospholipid bilayer of the liposomes. Specifically, this patent application discloses a nanostructure having a liposome core composed of a lipid bilayer and oligonucleotides located outside the liposome core, wherein an immunostimulant is bound to the lipid bilayer; the oligonucleotides form an oligonucleotide shell and are anchored to the surface of the liposome core through conjugation with a linker molecule; the linker molecule is tocopherol or cholesterol; and all of the oligonucleotides have 5'-ends exposed on the outer surface of the nanostructure. However, there exists a wide variety of oligonucleotides, and the structures formed by different types of oligonucleotides with liposomes, as well as the direct interactions between them, also vary significantly.

Therefore, the direct interaction between specific adjuvants and liposomes, as well as the resulting immune effects, remains an urgent issue to be investigated.

### Summary of the Invention

In view of the above problems, it is an object of the present invention to provide an immunological adjuvant composition, a preparation method and use thereof. The immunological adjuvant composition is capable of eliciting an immune response against a human herpesvirus and/or a hepatitis B virus in a mammal, and inducing a relatively strong level of cellular immune response.

### Definitions:

Unless otherwise defined, all scientific and technical terms used herein have the same meanings as understood by those of ordinary skill in the art. With regard to the definitions of terms in the art, those skilled in the art may specifically refer to *Current Protocols in Molecular Biology* (Ausubel).

Although the present invention discloses numerical ranges and approximate parameters in a broad sense, the values set forth in the specific examples are described as accurately as possible. However, any numerical values inherently contain a certain error, which results from the standard deviations existing in their respective measurements. In addition, all ranges disclosed herein shall be construed to cover any and all subranges encompassed therein. For example, a stated range of "2 to 40" shall be considered to include any and all subranges between (and inclusive of) the minimum value of 2 and the maximum value of 40, that is, all subranges starting from the minimum value of 2 or higher, such as 2 to 6.1, and ending at the maximum value of 40 or lower, such as 5.5 to 40. In addition, any reference cited as "incorporated herein" shall be understood to be incorporated in its entirety.

The term "or" used herein may be used interchangeably with the term "and/or", unless the context clearly indicates otherwise.

The term "immunological adjuvant composition" used herein may be used interchangeably with "combined immunological adjuvant" and "immunological adjuvant combination", and refers to a combination of at least one drug and an optional pharmaceutically acceptable excipient or auxiliary material, which are combined together to achieve a specific purpose.

The term "mammal" used herein refers to humans or other animals, such as wild animals (e.g., herons, storks, cranes, etc.), domestic animals (e.g., ducks, geese, etc.), or laboratory animals (e.g., orangutans, monkeys, rats, mice, rabbits, guinea pigs, marmots, ground squirrels, etc.).

The term "pseudo-binary mixture" used herein refers to a complex formed by the association of an adjuvant, such as saponin, with a sterol, such as cholesterol, in a liposome via van der Waals forces or hydrophobic interactions.

The above object of the present invention is achieved by the following technical solutions:
In a first aspect, the present invention provides an immunological adjuvant composition comprising an immune activator and a liposome for loading the immune activator, wherein the immune activator comprises a saponin and a CpG oligodeoxynucleotide.

Preferably, the liposome comprises a phospholipid and a sterol.

Further preferably, the phospholipid is one or more selected from the group consisting of dioleoyl phosphatidylcholine, egg yolk phosphatidylcholine, phosphocholine, or natural phospholipid derivatives; more preferably, the phospholipid is dioleoyl phosphatidylcholine (DOPC).

Further preferably, the sterol is one or more selected from the group consisting of cholesterol, stigmasterol, or ergosterol; more preferably, the sterol is cholesterol.

Preferably, the liposome has a phospholipid bilayer comprising an outer hydrophilic surface and an inner hydrophobic region. The saponin is embedded in the inner hydrophobic region, and the CpG oligodeoxynucleotide is associated with the outer hydrophilic surface. Further preferably, the CpG oligodeoxynucleotide is associated with the outer hydrophilic surface via van der Waals forces and hydrophobic interactions.

Preferably, the sterol is embedded in the inner hydrophobic region of the phospholipid bilayer.

Preferably, the saponin and the sterol form a pseudo-binary mixture of wormlike micelle assemblies. Further preferably, the pseudo-binary mixture is formed by both the hydrophobic interactions between the hydrophobic groups of the sterol in the liposome and the triterpene moieties of the saponin, and the hydrogen bonding between the hydrophilic groups of the sterol and the glycosyl moieties of the saponin.

Preferably, the CpG oligodeoxynucleotide has two or more copies of the 5'-TTCGTT-3' motif or the 5'-TCGTCGTCG-3' motif.

Preferably, the CpG oligodeoxynucleotide comprises or consists of a sequence selected from the following:
(1) a nucleotide sequence as set forth in any one of SEQ ID NO: 1 to SEQ ID NO: 13;
(2) a nucleotide sequence having at least 90%, 91%, 92%, 93%, 94%, 95%, 96%, 97%, 98%, 99% or more identity to the nucleotide sequence as set forth in any one of SEQ ID NO: 1 to SEQ ID NO: 13;
(3) a nucleotide sequence having one or more, such as 2, 3, 4 or 5, nucleotide substitutions, deletions, or insertions relative to the nucleotide sequence as set forth in any one of SEQ ID NO: 1 to SEQ ID NO: 13.

Further preferably, the CpG oligodeoxynucleotide comprises or consists of the nucleotide sequence as set forth in SEQ ID NO: 1, SEQ ID NO: 2 or SEQ ID NO: 3.

Further preferably, the CpG oligodeoxynucleotide comprises or consists of the nucleotide sequence as set forth in SEQ ID NO: 1.

Preferably, the saponin is one or more selected from the group consisting of quillaja saponin, ginsenoside, platycodin, astragaloside, notoginsenoside, glycyrrhizin, albiziae saponin, ophiopogonin, saikosaponin, or panax japonicus saponin.

Preferably, the quillaja saponin is one or more selected from the group consisting of QS-7, QS-17, QS-18, or QS-21; further preferably, the quillaja saponin is QS-21.

Preferably, the weight ratio of the phospholipid to the sterol is (20-4000): (50-1000), preferably (20-2000): (50-500).

Preferably, the weight ratio of the combination of the liposome and the saponin to the CpG oligodeoxynucleotide is (0.1-5): (0.5-120), preferably (1: 5)-20, and more preferably 1: 10.

Preferably, the weight ratio of the saponin to the liposome is (0.1-5): (10-150), preferably (0.2-5): (15-150).

Preferably, the immunological adjuvant composition is in the form of nanoparticles; further preferably, the particle size of the nanoparticles is 20-120 nm.

Preferably, the immunological adjuvant composition further comprises an antigen, a fragment thereof, a variant thereof, or a mixture of at least two thereof.

Preferably, the antigen is one or more selected from the group consisting of a hepatitis A, B, C or E virus antigen, a human herpesvirus antigen, a human immunodeficiency virus antigen, a varicella-zoster virus antigen, a human cytomegalovirus antigen, a respiratory syncytial virus antigen, a human papillomavirus antigen, an influenza virus antigen or a *Mycobacterium tuberculosis* antigen; further preferably, the antigen is a hepatitis B virus antigen or a herpesvirus antigen.

Preferably, the hepatitis B virus antigen is a hepatitis B virus surface antigen (HBsAg) and a hepatitis B virus core antigen (HBcAg).

Preferably, the hepatitis B surface antigen comprises or consists of an amino acid sequence selected from the following:
(1) an amino acid sequence as set forth in SEQ ID NO: 14;
(2) an amino acid sequence having at least 90%, 91%, 92%, 93%, 94%, 95%, 96%, 97%, 98%, 99% or more identity to the amino acid sequence as set forth in SEQ ID NO: 14;
(3) an amino acid sequence having one or more amino acid substitutions, deletions or insertions relative to the amino acid sequence as set forth in SEQ ID NO: 14.

Further preferably, the hepatitis B surface antigen comprises or consists of the amino acid sequence as set forth in SEQ ID NO: 14.

Preferably, the hepatitis B core antigen comprises or consists of an amino acid sequence selected from the following:
(1) an amino acid sequence as set forth in SEQ ID NO: 15;
(2) an amino acid sequence having at least 90%, 91%, 92%, 93%, 94%, 95%, 96%, 97%, 98%, 99% or more identity to the amino acid sequence as set forth in SEQ ID NO: 15;
(3) an amino acid sequence having one or more amino acid substitutions, deletions or insertions relative to the amino acid sequence as set forth in SEQ ID NO: 15.

Further preferably, the hepatitis B core antigen comprises or consists of the amino acid sequence as set forth in SEQ ID NO: 15.

Preferably, the herpesvirus antigen is the herpesvirus gE protein.

Preferably, the herpesvirus gE protein comprises or consists of an amino acid sequence selected from the following:
(1) an amino acid sequence as set forth in SEQ ID NO: 16;
(2) an amino acid sequence having at least 90%, 91%, 92%, 93%, 94%, 95%, 96%, 97%, 98%, 99% or more identity to the amino acid sequence as set forth in SEQ ID NO: 16;
(3) an amino acid sequence having one or more amino acid substitutions, deletions or insertions relative to the amino acid sequence as set forth in SEQ ID NO: 16.

Further preferably, the herpesvirus gE protein comprises or consists of the amino acid sequence as set forth in SEQ ID NO: 16.

Preferably, the weight ratio of the herpesvirus antigen to the combination of the liposome and the saponin to the CpG oligodeoxynucleotide is (0.1-5): (0.1-5): (0.5-120), preferably (1-2): 1: (5-20), and more preferably 1: 1: 10.

Preferably, the weight ratio of the hepatitis B virus antigen to the combination of the liposome and the saponin to the CpG oligodeoxynucleotide is (5-100): (0.1-5): (0.5-120), preferably (15-50): 1: (5-20), and more preferably 30: 1: 10.

In a second aspect, the present invention provides a method for preparing the immunological adjuvant composition according to the first aspect of the present invention, comprising the following steps:
dissolving the saponin in an aqueous solution, adding the liposome thereto, performing a first stirring, then adding the CpG oligodeoxynucleotide, and performing a second stirring to obtain the immunological adjuvant composition.

Preferably, the stirring speed of each of the first stirring and the second stirring is independently 50-150 rpm, preferably 50-100 rpm.

Preferably, the aqueous solution comprises a phosphate and sodium chloride.

Further preferably, the concentration of phosphate in the aqueous solution is 10-50 mmol/L, preferably 20 mmol/L; the concentration of sodium chloride in the aqueous solution is 100-350 mmol/L, preferably 150 mmol/L.

Preferably, the method further comprises a step of adding an antigen after the second stirring.

In a third aspect, the present invention provides an immunological kit comprising the immunological adjuvant composition according to the first aspect of the present invention.

In a fourth aspect, the present invention provides use of the immunological adjuvant composition according to the first aspect of the present invention or the immunological kit according to the third aspect of the present invention in the manufacture of the following products:
(1) a medicament for the prevention and/or treatment of a human herpesvirus and/or hepatitis B virus infection or a disease associated therewith; preferably, the medicament is a vaccine;
(2) a kit for diagnosing a human herpesvirus and/or hepatitis B virus infection; or
(3) an immunogen for developing an antibody against human herpesvirus and/or hepatitis B virus.

Preferably, the human herpesvirus is one or more selected from the group consisting of a varicella-zoster virus, a herpes simplex virus type 1 (HSV1), or a herpes simplex virus type 2 (HSV2).

The present invention has at least the following beneficial effects:
In the immunological adjuvant composition provided by the present invention, an interaction can occur among the saponin such as QS21, the CpG oligodeoxynucleotide, and the liposome. QS21 is embedded in the inner hydrophobic region of the phospholipid bilayer and forms a pseudo-binary mixture of wormlike micelle assemblies with a sterol such as cholesterol. Such assemblies having a pseudo-binary mixture structure are formed by both the hydrophobic interactions between the hydrophobic groups of cholesterol in the liposome and the triterpene moieties of QS21, and the hydrogen bonding between the hydrophilic groups of cholesterol and the glycosyl moieties of QS21. The hydrophobic interactions and hydrogen bonding can enhance the binding efficiency between QS21 and the liposome and drug loading capacity, thereby improving the stability of the QS21 liposome.

In the immunological adjuvant composition provided by the present invention, the CpG oligodeoxynucleotide is negatively charged, and van der Waals forces and hydrophobic interactions exist between it and phospholipid molecules such as DOPC, such that CpG can directly associate with the liposome at its surface, enhancing the binding efficiency between CpG and the liposome and the drug loading capacity, thereby improving the stability of the CpG-QS21 liposome.

The immunological adjuvant composition provided by the present invention can elicit an immune response against a human herpesvirus and/or hepatitis B virus in a mammal, and can induce a relatively strong level of cellular immune response. Specifically, the immunological adjuvant composition provided by the present invention can elicit relatively high levels of gE antigen-specific IFN-γ and high titers of gE antigen-specific IgG/IgG1/IgG2a in a mammal, and the induced gE antigen-specific IFN-γ and titers of gE antigen-specific IgG/IgG1/IgG2a remain at relatively high levels even after a prolonged period. The immunological adjuvant composition provided by the present invention can effectively reduce the HBsAg level in HBV-infected model mice, and can induce high levels of HBsAg-specific and HBcAg-specific IFN-γ.

By simultaneous loading of both QS21 and the CpG oligodeoxynucleotide using the liposome, they have a favorable synergistic effect in promoting cellular immunity. The immunological adjuvant composition of the present invention can induce a stronger level of immune response compared to the commercially available vaccine Shingrix.

### Brief Description of the Drawings

Hereinafter, the embodiments of the present invention will be described in detail with reference to the accompanying drawings, wherein:
FIG. 1 is a cryo-electron microscopy image of the CpG-QS21 liposomal nanocomposite formulation prepared in Example 2 of the present invention;
FIG. 2 is a schematic structural diagram of the CpG-QS21 liposome;
FIG. 3 is a particle size diagram of the CpG-QS21 liposomal nanocomposite formulation prepared in Example 2 of the present invention;
FIG. 4 shows the effects of CpG oligodeoxynucleotides in different ratios on the spot counts of herpesvirus gE antigen-specific IFN-γ-secreting T lymphocytes in Example 3 of the present invention;
FIG. 5 shows the effects of CpG oligodeoxynucleotides in different ratios on the spot counts of herpesvirus gE antigen-specific IFN-γ-secreting T lymphocytes in Example 4 of the present invention;
FIG. 6 shows the effects of different CpG oligodeoxynucleotides on the levels of herpesvirus gE antigen-specific IgG and its subtypes, as well as the IgG2a/IgG1 ratio in Example 4 of the present invention;
FIG. 7 shows the effects of herpesvirus gE antigen in different ratios on the spot counts of gE-specific IFN-γ-secreting lymphocytes from splenocytes in Example 5 of the present invention;
FIG. 8 shows the effects of herpes zoster vaccines on the spot counts of gE-specific IFN-γ-secreting lymphocytes from mouse splenocytes in Example 6 of the present invention;
FIG. 9 shows the effects of herpes zoster vaccines on the levels of gE-specific IgG and its subtypes, as well as the IgG2a/IgG1 ratio in mouse serum in Example 6 of the present invention;
FIG. 10 shows the effects of hepatitis B vaccines on HBsAg levels in mouse serum at different weeks post-immunization in Example 7 of the present invention;
FIG. 11 shows the effects of hepatitis B vaccines on the spot counts of gE-specific IFN-γ-secreting lymphocytes from mouse splenocytes in Example 7 of the present invention.

### Best Modes for Carrying Out the Invention

The present invention will be further described in detail below with reference to the specific embodiments, and the examples provided are merely intended to illustrate the present invention, and not to limit the scope of the present invention.

### Example 1: Preparation of Biological Materials

1. HBsAg stock solution: The amino acid sequence of the HBsAg protein used in the present invention is SEQ ID NO: 14, which corresponds to SEQ ID NO: 1 in Chinese Patent CN108330145B. The HBsAg antigen protein was prepared by recombinant yeast cells carrying the HBsAg gene, and a crude HBsAg fermentation broth was obtained; see Chinese Patent CN108330145B, paragraphs 0044 to 0091 for the specific preparation method. Cell lysis, filtration, and column chromatography were performed to obtain the HBsAg stock solution; see Chinese Patent CN108330145B, paragraphs 0092 to 0136 for the specific purification method.
2. HBcAg stock solution: The amino acid sequence of the HBcAg protein used in the present invention is SEQ ID NO: 15, which corresponds to SEQ ID NO: 1 in Chinese Patent CN104673760B. The HBcAg antigen protein was prepared by recombinant yeast cells carrying the HBcAg gene, and a crude HBcAg fermentation broth was obtained; see Chinese Patent CN104673760B, paragraphs 0049 to 0068 for the specific preparation method. Cell lysis, filtration, and column chromatography were performed to obtain the HBcAg stock solution; see Chinese Patent CN104673760B, paragraphs 0069 to 0080 for the specific purification method.
3. gE stock solution: The amino acid sequence of the gE protein is SEQ ID NO: 16, which corresponds to SEQ ID NO: 1 in Chinese Patent CN112972671A. The nucleic acid sequence of the gE protein was subjected to codon optimization and PCR amplification, followed by ligation into the target vector and transformation. The target plasmid was obtained upon screening and then transfected into CHO cells. After fermentation, the product was filtered and purified to obtain the gE stock solution; see Thomsson E, Persson L, et al., Journal of Virological Methods, 20, 2011, Vol. 175, No. 1, pp. 53-59 for the specific preparation method.
4. PBS solution: Purchased from Hyclone, Cat. No. SH30256.01.
5. Serum-free medium: Purchased from Dakewe Biotech Co., Ltd., Cat. No. 6015012.
6. QS21: Purchased from BRENNTAG, CAS. NO. A010-023.
7. Preparation method of CpG oligodeoxynucleotide raw material: The CpG oligodeoxynucleotide was prepared by a conventional solid-phase phosphoramidite triester chemical synthesis method. Starting from the 3' end, deprotection was performed first, followed by activation, coupling, oxidation, and capping to obtain a crude DNA fragment. Finally, post-synthesis treatments were carried out, including cleavage, deprotection, purification, and quantification; see Patent CN200810004736.0, paragraphs 0063 to 0064 for the specific preparation method. The CpG oligodeoxynucleotide used in the present invention was CpG T1, and the specific sequence is shown in Table 1.
8. Shingrix vaccine: Purchased from GlaxoSmithKline Biologicals SA.

**Table 1. Specific sequences of CpG oligodeoxynucleotides**

| Type of CpG Oligodeoxynucleotide | CpG Oligodeoxynucleotide | Sequence |
|---|---|---|
| Type B | CpG T1 | TCGTTCGTTCGTTCGTTCGTT (SEQ ID NO: 1) |
| | CpG T2 | TCGTTCGTTCGTTCG TTCGTTCGTT (SEQ ID NO: 2) |
| | CpG T3 | TCGTCGTCGTCGTCGTCGTCG (SEQ ID NO: 3) |
| | CpG 1018 | TGACTGTGAACGTTCGAGATGA (SEQ ID NO: 4) |
| | CpG 7909 | TCGTCGTTTTGTCGTTTTGTCGTT (SEQ ID NO: 5) |
| | CpG 1826 | TCCATGACGTTCCTGACGTT (SEQ ID NO: 6) |
| | CpG 684 | TCGACGTTCGTCGTTCGTCGTTC (SEQ ID NO: 7) |
| | CpG 1668 | TCC ATG ACG TTC CTG ATGCT (SEQ ID NO: 8) |
| | CpG D2 | TGTCGTCGTCGTTTGTCGTTTGTCGTT (SEQ ID NO: 9) |
| Type A | CpG 2216 | GGGGGACGATCGTCGGGGGG (SEQ ID NO: 10) |
| | ODN 2336 | GGGGACGACGTCGTGGGGGGG (SEQ ID NO: 11) |
| Type C | ODN 2395 | TCGTCGTTTCGCGCGCGCCG (SEQ ID NO: 12) |
| | ODNM362 | TCGTCGTTCGTTCGTCGAACGACGTTTGAT (SEQ ID NO: 13) |

### Example 2: Preparation and Detection of Liposomal Nanocomposite Formulations

### 1. Preparation of Liposomal Nanocomposite Formulation

### (1) Preparation of Blank Liposome

Dioleoyl phosphatidylcholine and cholesterol were dissolved in an organic solvent at a weight ratio of 1000: 250, and the organic solvent was removed by rotary evaporation. The rotary evaporation temperature was set to 50°C; the temperature of the low-temperature circulation device was set to -10°C; the vacuum degree for rotary evaporation was maintained at 20-120 hPa, and the rotational speed was maintained at 30-180 rpm, so as to form a lipid thin film on the wall of the rotary evaporator flask. The lipid thin film was dissolved in an aqueous solution and hydrated to form a lipid suspension, which was then homogenized 6 times at a controlled homogenization pressure of 150 bar. The obtained lipid solution was extruded 6 times through two polycarbonate filter membranes, wherein the pore size of the first polycarbonate filter membrane was 200 nm and the pore size of the second polycarbonate filter membrane was 100 nm, thereby obtaining the blank liposome.

Here, the organic solvent was absolute ethanol, and the aqueous solution was a solution of a phosphate and sodium chloride, wherein the concentration of phosphate (i.e., sodium dihydrogen phosphate and disodium hydrogen phosphate) was 20 mmol/L and the concentration of sodium chloride was 150 mmol/L.

### (2) Preparation of CpG-QS21 Liposome

QS21 was dissolved in an aqueous solution, and the blank liposome was added thereto and stirred uniformly at a controlled stirring speed of 50-150 rpm, so as to obtain the QS21 liposome. Then, the CpG oligodeoxynucleotide was added and stirred uniformly with the QS21 liposome at a controlled stirring speed of 50-150 rpm, so as to obtain the CpG-QS21 liposome. Here, the aqueous solution was a solution of a phosphate and sodium chloride, wherein the concentration of phosphate was 20 mmol/L and the concentration of sodium chloride was150 mmol/L. The weight ratio of QS21 to the liposome was kept constant at 50: 1250, and they were combined and referred to as the QS21 liposome.

The CpG-QS21 liposome prepared in this example was mixed with the desired antigen stock solution for injection use in the following examples.

### 2. Detection of Liposomal Nanocomposite Formulation

### (1) Determination of Encapsulation Efficiency of QS21

The encapsulation efficiency of the CpG-QS21 liposome was determined by high-performance liquid chromatography. The liposomal nanocomposite formulation used for this determination had a ratio for human vaccine. Each dose was 0.5 mL, containing 1300 µg of the QS21 liposome (including 50 µg of QS21), 500 µg of CpG, and 100 µg of gE antigen.

Detection method for encapsulation efficiency: The encapsulation efficiency of QS21 was determined by high-performance liquid chromatography, wherein octadecylsilane-bonded silica gel was used as the packing material; mobile phase A was formic acid-water (1: 1000) and mobile phase B was formic acid-acetonitrile (1: 1000) for linear gradient elution; the flow rate was 1.0 mL per minute; the column temperature was 40°C; the injection volume was 100 µL; and the detector was CAD (nebulization temperature: 50°C; acquisition frequency: 10 Hz).

The encapsulation efficiency was calculated as follows: $Encapsulation efficiency \left(%\right) = \frac{100 {A}_{L}}{100 {A}_{L} + 20 {A}_{F}} \times 100 % ,$ wherein A_{L} is the sum of the peak areas of QS-21a and QS-21b encapsulated in the liposome;
A_{F} is the sum of the peak areas of free QS-21a and QS-21b;
100 represents the dilution factor of the encapsulated portion solution;
20 represents the dilution factor of the free portion solution.

### (2) Microscopic Morphology and Particle Size Determination of Liposomal Nanocomposite Formulation

The microscopic morphology of the CpG-QS21 liposome was observed using cryo-electron microscopy.

The average particle size of the CpG-QS21 liposome was determined by dynamic laser light scattering method. The dynamic laser light scattering method was carried out as follows: The measuring cell was rinsed once with filtered double-distilled water. The sample was subjected to water-bath ultrasonication (100 W, ultrasonication performed 5-7 times), and then filtered through a 0.22 m syringe filter (Pall Corporation). A 1 m sample was taken and placed into the measuring cell (the height of the sample in the measuring cell was 10 mm-15 mm). In accordance with the instrument manual, the cover of the sample compartment was opened, the measuring cell was placed in position (with the side marked with a V facing the operator), and the "Start" button was clicked to initiate the measurement.

### 3. Detection Results

**Table 2. Encapsulation efficiency of QS21 in the CpG-QS21 liposome**

| QS21 (µg/mL) | 10 | 20 | 50 | 100 | 150 | 200 | 400 |
|---|---|---|---|---|---|---|---|
| Encapsulation efficiency (%) | 94.3 | 92.7 | 92.4 | 92.6 | 91.8 | 89.9 | 84.4 |

The encapsulation efficiency of QS21 in the CpG-QS21 liposome, as determined by high-performance liquid chromatography, is shown in Table 2. When the concentration of QS21 was increased to 100 µg/mL, a further increase in the QS21 content resulted in a declining trend in the encapsulation efficiency of QS21. However, when the QS21 content was 10-200 µg/mL, the encapsulation efficiency remained above the industry-recognized standard level (85%), which may be related to the encapsulation loading capacity of the liposome.

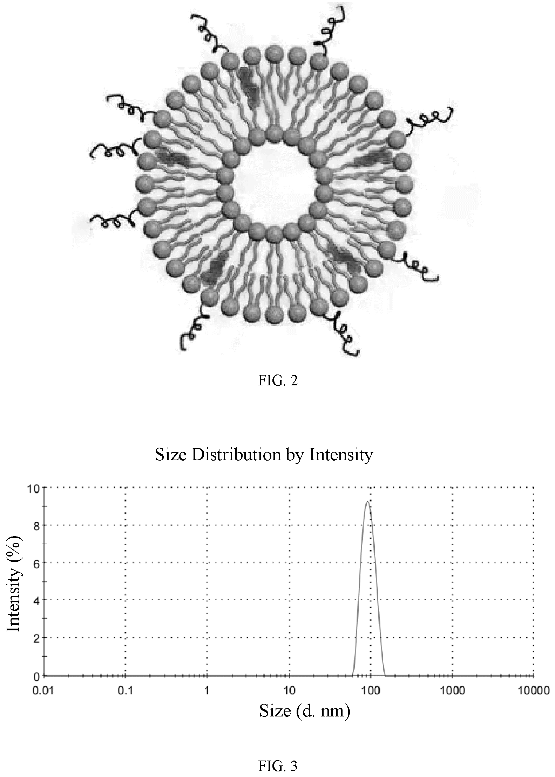

The particle size of the CpG-QS21 liposome, as determined by dynamic laser light scattering method, is shown in FIG. 3, and its average particle size was 93.08 nm.

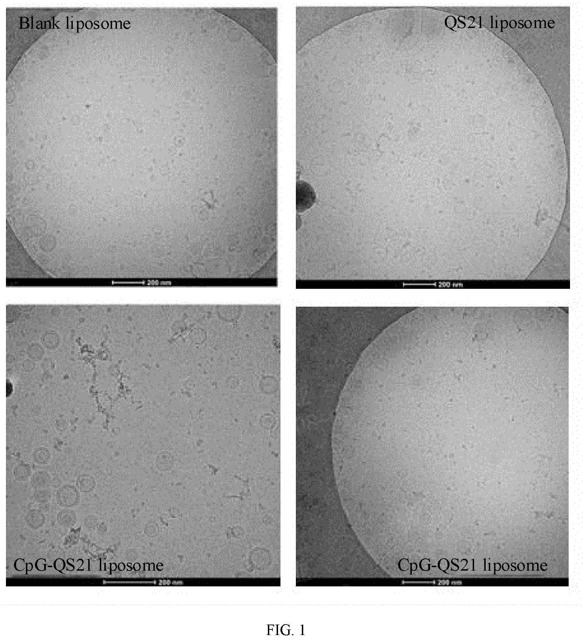

The microscopic morphological characterization of the CpG-QS21 liposome is shown in FIG. 1. The schematic structural diagram of the CpG-QS21 liposome is shown in FIG. 2. It can be seen that the blank liposome, the QS21 liposome, and the CpG-QS21 liposome all have a phospholipid bilayer structure, with each phospholipid molecule having a hydrophilic head and a lipophilic tail. The two layers of phospholipid molecules are arranged oppositely, forming an outer hydrophilic surface and an inner hydrophobic region. Cholesterol is located in the inner hydrophobic region, and QS21 is embedded in the same region to form a pseudo-binary mixture of wormlike micelle assemblies with cholesterol. Such assemblies having a pseudo-binary mixture structure are formed by both the hydrophobic interactions between the hydrophobic moieties of cholesterol in the liposome and the triterpene moieties of QS21, and the hydrogen bonding between the hydrophilic moieties of cholesterol and the glycosyl moieties of QS21. The hydrophobic interactions and hydrogen bonding can enhance the binding efficiency between QS21 and liposome and drug loading capacity, thereby improving the stability of the QS21 liposome.

Since the CpG oligodeoxynucleotide is negatively charged, and van der Waals forces and hydrophobic interactions exist between it and the liposome, the CpG oligodeoxynucleotide associates with the phospholipid molecule DOPC on the liposome's surface. Due to the van der Waals forces and hydrophobic interactions between CpG and the phospholipid molecule DOPC, CpG can directly associate with the liposome at its surface, enhancing the binding efficiency between CpG and liposome and drug loading capacity, thereby improving the stability of the CpG-QS21 liposome.

Patent application CN106535876A discloses a liposomal nanostructure having a liposome core composed of a lipid bilayer and oligonucleotides containing Class B CpG motifs located outside the liposome core, wherein all the oligonucleotides form an oligonucleotide shell, and the oligonucleotide shell is anchored to the surface of the liposome core through conjugation with a linker molecule. The linker molecule is tocopherol or cholesterol, and the surface of the liposome core is the surface of the hydrophobic region of the liposome. That is to say, for the liposome disclosed in patent application CN106535876A, the oligonucleotides need to enter the interior of the phospholipid bilayer of the liposome through gaps among phospholipid molecules on the outer surface, so as to be anchored to the surface of the liposome's hydrophobic region via the linker molecule. Obviously, the linker molecule is located in the core region of the liposome, i.e., its hydrophobic region. The oligonucleotides must pass through the gaps among phospholipid molecules on the outer surface to enter the interior of the phospholipid bilayer of the liposome, thereby contacting the linker molecule and being further anchored to the surface of the liposome's hydrophobic region. Compared with the liposome structure of patent application CN106535876A, on the one hand, in the liposome structure of the present invention, CpG can directly associate with the liposome at its surface, which is more direct and efficient, and can significantly enhance the binding efficiency of CpG; on the other hand, in the liposome structure of the present invention, the association between CpG and the liposome relies on inherent van der Waals forces, and effective binding can be achieved without any linker molecule, which further enhances the binding efficiency between CpG and the liposome.

### Example 3: Preliminary Screening Experiment of CpG-QS21 Liposomal Vaccines

1. Experimental animals: Female 6-week-old C57BL/6 mice, n = 60, purchased from Shanghai Lingchang Biotechnology Co., Ltd., with the animal license No. SCXK(Hu) 2018-0003.
2. Animal grouping: The detailed grouping is shown in Table 3. The injection volume per dose was 100 µL per mouse. Before injection, the components listed in the table below for each group were dissolved in PBS solution and diluted to 100 µL. The doses of CpG and QS21 liposome in the CpG-QS21 liposome for each group are shown in the table below.

**Table 3. Grouping of the SM21053 animal experiment**

| Group | Number (mice) | Component (µg per mouse) | | |
|---|---|---|---|---|
| | | gE antigen | CpG-QS21 liposome | |
| | | | QS21 liposome | CpG |
| A | 5 | 2 | 2 | 2 |
| B | 5 | 2 | 2 | 4 |
| C | 5 | 2 | 2 | 10 |
| D | 5 | 2 | 2 | 20 |
| E | 5 | 2 | 2 | 50 |
| F | 5 | 2 | 2 | 100 |
| G | 5 | 2 | 1 | 2 |
| H | 5 | 2 | 1 | 4 |
| I | 5 | 2 | 1 | 10 |
| J | 5 | 2 | 1 | 20 |
| K | 5 | 2 | 1 | 50 |
| L | 5 | 2 | 1 | 100 |

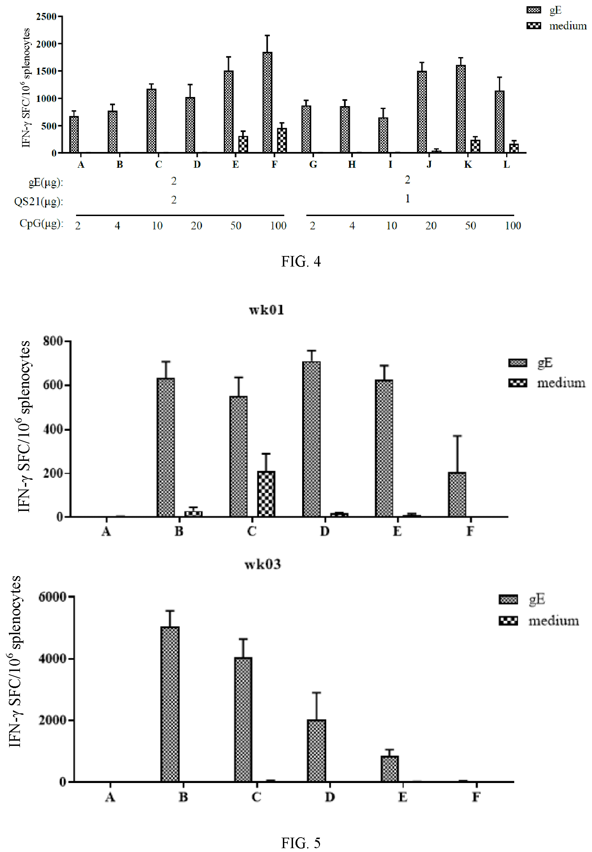

3. Experimental procedure: Intramuscular immunization was performed, with a dose of 100 µL per mouse for each group, and a single immunization was carried out in total. At week 1 (wk01, one week after the primary immunization), the spot counts of gE-specific IFN-γ-secreting T lymphocytes from splenocytes were determined. Before spot counting, Groups A to L were injected with serum-free medium containing a gE-specific stimulating peptide pool. Meanwhile, a medium negative control group (medium group) was set for each group. For the medium group, splenocytes were isolated from unimmunized mice and injected with an equal volume of serum-free medium without the aforementioned stimulating peptide pool.
4. Experimental results: The ELISpot assay was used to determine the spot counts of gE-specific IFN-γ-secreting T lymphocytes from splenocytes, and the results are shown in FIG. 4. The results indicate that:
a) Comparison among Groups A to F, immunized with 2 µg of gE antigen combined with CpG-QS21 liposomes containing 2 µg of QS21 liposome and CpG at different doses (2-100 µg), shows that the induced levels of gE antigen-specific IFN-γ exhibited a continuous upward trend with the increasing CpG doses. In contrast, Groups E (comprising 50 µg CpG) and F (comprising 100 µg CpG) with high-dose CpG induced a certain level of non-specific IFN-γ response in mouse splenocytes, and the status of splenocytes exhibited a tendency toward abnormal enlargement. In addition, the splenocytes from Group C, immunized with 10 µg of CpG, secreted a relatively high level of gE antigen-specific IFN-γ (approximately 1200 SFC/10⁶ splenocytes), with normal splenocyte status.
b) Comparison among Groups G to L, immunized with 2 µg of gE antigen combined with CpG-QS21 liposomes containing 1 µg of QS21 liposome and CpG at different doses (2-100 µg), shows that Groups K (50 µg CpG) and L (100 µg CpG) with high-dose CpG also induced a certain level of non-specific IFN-γ response in mouse splenocytes, and the splenocyte status also exhibited a tendency toward abnormal enlargement, similar to the observations in Groups E and F. In addition, splenocytes from Group J, immunized with 20 µg of CpG, secreted the highest level of gE antigen-specific IFN-γ (approximately 1500 SFC/10⁶ splenocytes), with normal splenocyte status.
c) In the CpG-QS21 liposomes, the combination of different doses of QS21 liposomes (1 µg and 2 µg) and CpG could all induce relatively strong levels of gE antigen-specific IFN-γ.

Based on the above results, in CpG-QS21 liposomes, when the dose of QS21 liposome is 1 µg, the preferred weight ratio of QS21 liposome to CpG is 1: 20; when the dose of QS21 liposome is 2 µg, the preferred weight ratio of QS21 liposome to CpG is 1: 5.

**Table 4. Positive conversion rate (%) of gE-specific IFN-γ secreted by splenocytes**

| Group | A | B | C | D | E | F |
|---|---|---|---|---|---|---|
| Positive conversion rate | 100 (5/5) | 100 (5/5) | 100 (5/5) | 100 (5/5) | 100 (5/5) | 80 (4/5) |

| Group | G | H | I | J | K | L |
|---|---|---|---|---|---|---|
| Positive conversion rate | 100 (5/5) | 100 (5/5) | 100 (5/5) | 100 (5/5) | 100 (5/5) | 100 (5/5) |

### Example 4: Screening Experiment for Vaccines Containing CpG-QS21 Liposomal Nanocomposite Formulation

1. Experimental animals: Female 4-5-week-old C57BL/6 mice, n = 72, purchased from Shanghai Lingchang Biotechnology Co., Ltd., with the animal license No. SCXK(Hu) 2018-0003.
2. Animal grouping: The detailed grouping is shown in Table 5. The injection volume per dose was 100 µL per mouse. Before injection, the components listed in the table below for each group were dissolved in PBS solution and diluted to 100 µL. The doses of CpG and QS21 liposome in the CpG-QS21 liposome for each group are shown in the table below. Group A served as the negative control and was injected with 100 µL of PBS containing 2 µg of gE antigen per mouse; Group F was administered the commercially available vaccine Shingrix, in which the antigen dose was 2 µg per mouse, and the doses of both QS21 liposome and MPL were 2 µg per mouse.

**Table 5. Grouping of the SM21056 animal experiment**

| Group | Number (mice) | Component (µg per mouse) | | | |
|---|---|---|---|---|---|
| | | gE antigen | CpG-QS21 liposome | | Commercially available vaccine Shingrix |
| | | | QS21 liposome | CpG | |
| A | 12 | 2 | | | |
| B | 12 | 2 | 2 | 20 | |
| C | 12 | 2 | 2 | 40 | |
| D | 12 | 2 | 1 | 10 | |
| E | 12 | 2 | 1 | 20 | |
| F | 12 | | | | 2 µg gE antigen + 2 µg QS21 liposome + 2 µg MPL |

3. Experimental procedure: Intramuscular immunization was performed with two injections administered at week 0 (wk00) and 2 (wk02), respectively. Groups A to E were injected with 100 µL per mouse, and Group F was injected with 20 µL per mouse. At week 1 (wk01), half of the mice were sampled for determination; the remaining half received the secondary immunization at week 2 (wk02), and the determinations were performed at week 3 (wk03), including the determination of spot counts of IFN-γ-secreting T lymphocytes from splenocytes and the determination of gE antigen-specific antibodies in serum. Before spot counting, Groups A to F were injected with serum-free medium containing a gE-specific stimulating peptide pool. Meanwhile, a medium negative control group (medium group) was set for each group. For the medium group, splenocytes were isolated from unimmunized mice and injected with an equal volume of serum-free medium without the aforementioned stimulating peptide pool.
4. Experimental Results

The ELISpot assay was used to determine the spot counts of gE-specific IFN-γ-secreting T lymphocytes from splenocytes, and the results are shown in FIG. 5. The results indicate that:
a) At week 1 (wk01, one week after the primary immunization), Group A, immunized with 2 µg of gE antigen, produced no detectable gE-specific IFN-γ level. Groups B and C, immunized with 2 µg of gE antigen combined with the CpG-QS21 liposome containing 2 µg of QS21 liposome at a QS21 liposome-to-CpG ratio of 1: 10 or 1: 20, both produced significant gE antigen-specific IFN-γ levels. However, Group C exhibited enhanced non-specific response and enlarged spleens in mice due to the high CpG dose (40 µg). In comparison, the dose condition of Group B was better (approximately 630 SFC per 10⁶ splenocytes). Groups D and E, immunized with 2 µg of gE antigen combined with the CpG-QS21 liposome containing 1 µg of QS21 liposome at a QS21 liposome-to-CpG ratio of 1: 10 or 1: 20, also produced gE antigen-specific IFN-γ levels equivalent to that of Group B, and Group D (1 µg QS21 liposome + 10 µg CpG) corresponded to a relatively higher IFN-γ level (approximately 700 SFC per 10⁶ splenocytes). Although the commercially available vaccine Shingrix group, immunized with the same antigen dose, could produce a gE antigen-specific IFN-γ level (approximately 200 SFC per 10⁶ splenocytes), the overall level was lower than those of Groups B to E.
b) At week 3 (wk03, one week after the secondary immunization), the gE antigen-specific IFN-γ levels produced by various immunized groups were elevated. The IFN-γ level of Group A, immunized with 2 µg of gE antigen, remained relatively low. For the immunization with 2 µg of gE antigen combined with the CpG-QS21 liposome at a QS21 liposome-to-CpG ratio of 1: 10 or 1: 20, under the identical dose of QS21 liposome, the cellular immune level induced at the QS21 liposome-to-CpG ratio of 1: 10 was higher than that at 1: 20 (i.e., Group B was superior to Group C, and Group D was superior to Group E). The gE antigen-specific IFN-γ levels induced by Group B and Group D reached 5000 SFC and 2000 SFC per 10⁶ splenocytes, respectively.

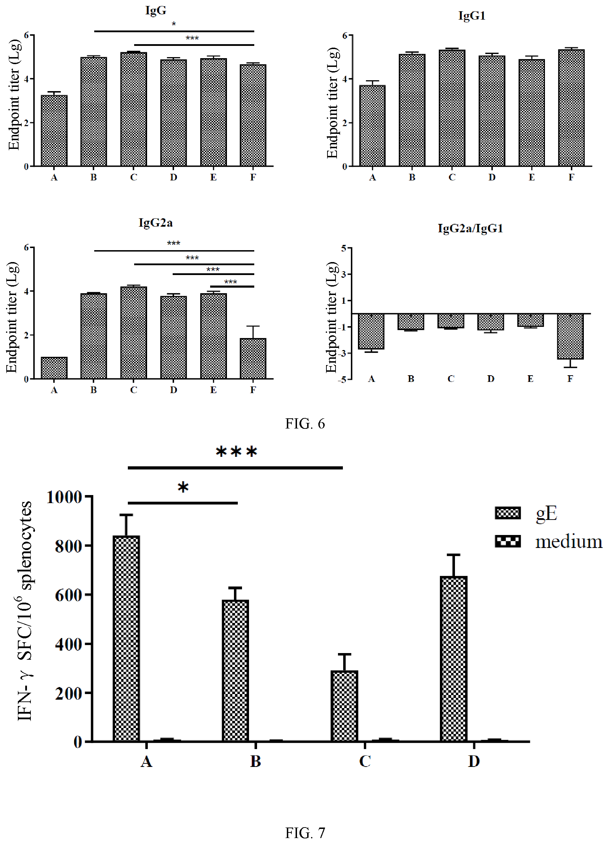

The levels of gE antigen-specific IgG/IgG1/IgG2a antibodies in mouse serum were determined by ELISA, and the results are shown in FIG. 6. The results indicate that:
At week 3 (wk03, one week after the secondary immunization), Group A, immunized with the antigen alone, could induce certain levels of IgG/IgG1 titers, whereas the IgG2a titer was relatively low. When the antigen was combined with the CpG-QS21 liposome at a QS21 liposome-to-CpG ratio of 1: 10 or 1: 20 (i.e., Groups B to E), the high titer levels of antigen-specific IgG/IgG1/IgG2a were induced (approximately 5.0 Lg, 5.0 Lg, and 4.0 Lg, respectively), with no significant differences among these groups. Immunization with the commercially available vaccine Shingrix group (Group F) at the same antigen dose could also induce relatively high titer levels of IgG/IgG1 (approximately 4.7 Lg and 5.3 Lg, respectively), but the IgG2a titer level was relatively low (only approximately 1.9 Lg), with significant differences when compared with Groups B to E (p < 0.001).

A comprehensive comparison among immunizations with the liposomal nanocomposite formulations with different ratios of CpG adjuvant to QS21 adjuvant shows that, the induced immune response level was more biased toward the Th1 pathway, whereas that induced by the commercially available vaccine Shingrix was more biased toward the Th2 pathway.

In conclusion, at the ratio of QS21 liposome to CpG at 1:10, the highest level of antigen-specific cellular immune response was induced, along with high titer levels of antigen-specific IgG/IgG1/IgG2a. These levels are superior to the cellular and humoral immune response levels induced by the commercially available vaccine Shingrix. Therefore, the ratio of QS21 liposome to CpG adjuvant in the CpG-QS21 liposomal nanocomposite formulation is preferably 1: 10.

### Example 5: Screening Experiment on Antigen Proportions for Vaccines Containing CpG-QS21 Liposomal Nanocomposite Formulation

1. Experimental animals: Female 6-week-old C57BL/6 mice, n = 32, purchased from Shanghai Lingchang Biotechnology Co., Ltd., with the animal license No. SCXK(Hu) 2018-0003.
2. Animal grouping: The detailed grouping is shown in Table 6. The injection volume per dose was 50 µL per mouse. Group D used the gE antigen from a different batch than those for Groups A to C. Before injection, the components listed in the table below for each group were dissolved in PBS solution and diluted to 50 µL.

**Table 6. Grouping of the SM22016 animal experiment**

| Group | Number (mice) | Component (µg per mouse) | | | |
|---|---|---|---|---|---|
| | | gE antigen 1 | gE antigen 2 | CpG-QS21 liposomal nanocomposite formulation | |
| | | | | QS21 liposome | CpG |
| A | 8 | 2 | | 1 | 10 |
| B | 8 | 1 | | 1 | 10 |
| C | 8 | 0.5 | | 1 | 10 |
| D | 8 | | 1 | 1 | 10 |

3. Experimental procedure: Intramuscular immunization was performed at 50 µL per mouse, with a single immunization in total. At week 1 (wk01, one week after the primary immunization), the spot counts of gE-specific IFN-γ-secreting T lymphocytes from splenocytes were determined. Before spot counting, Groups A to D were injected with serum-free medium containing a gE-specific stimulating peptide pool. Meanwhile, a medium negative control group (medium group) was set for each group. For the medium group, splenocytes were isolated from unimmunized mice and injected with an equal volume of serum-free medium without the aforementioned stimulating peptide pool.
4. Experimental results: Based on the study results of the ratio between the gE antigen and the CpG-QS21 liposomal nanocomposite formulation (see FIG. 7), the induced level of gE antigen-specific IFN-γ was dependent on the gE antigen dose after a single immunization with various doses of the gE antigens combined with the CpG-QS21 liposomal nanocomposite formulation (QS21 liposome: CpG = 1: 10). Among these, the combination of 1 µg of gE antigen and the CpG-QS21 liposomal nanocomposite formulation (the doses of QS21 liposome and CpG adjuvant were 1 µg and 10 µg, respectively) could induce a higher level of antigen-specific IFN-γ. Taking both antigen dose and cost into comprehensive consideration, the combination of 1 µg of gE antigen and CpG-QS21 liposome containing 1 µg of QS21 liposome and 10 µg of CpG was preferentially selected as the immunization dose for the efficacy test in mice. That is, the optimal ratio of gE antigen, QS21 liposome and CpG in the CpG-QS21 liposomal nanocomposite formulation was preliminarily determined to be 1: 1: 10.

### Example 6: Experimental Group Setting and Immunization Procedure for Herpes Zoster Vaccines

1. Experimental animals: Female 6-week-old C57BL/6 mice, n = 30, purchased from Shanghai Lingchang Biotechnology Co., Ltd., with the animal license No. SCXK(Hu) 2018-0003.
2. Animal grouping: The detailed grouping is shown in Table 7. The injection volume per dose was 100 µL. Before injection, the components listed in the table below for each group were dissolved in PBS solution and diluted to 100 µL.

**Table 7. Experimental animal grouping and administration doses**

| Group | Number (mice) | Component (µg per mouse) | | | | |
|---|---|---|---|---|---|---|
| | | gE antigen | QS21 liposome | CpG | CpG-QS21 liposomal nanocomposite formulation | Commercially available vaccine Shingrix |
| A | 5 | | | | | |
| B | 5 | 1 | | | | |
| C | 5 | 1 | 1 | | | |
| D | 5 | 1 | | 10 | | |
| E | 5 | 1 | | | 1 & 10* | |
| F | 5 | | | | | 1 µg gE antigen + 1 µg |
| | | | | | | QS21 liposome + 1 µg MPL |

| | | | | | | |
|---|---|---|---|---|---|---|
| * In the CpG-QS21 liposome, CpG is 10 µg, and QS21 liposome is 1 µg. | | | | | | |

3. Animal Immunization
Two repeated immunizations were performed at weeks 0 and 2 (wk00 and wk02) via intramuscular injection into the left or right posterior thigh.
4. Evaluation of Cellular Immune Effects of Herpes Zoster Vaccines
(1) Detection method: T lymphocyte spot counting assay was adopted. Before spot counting, Groups A to F were injected with serum-free medium containing a gE-specific stimulating peptide pool. Meanwhile, a medium negative control group (medium group) was set for each group. For the medium group, splenocytes were isolated from unimmunized mice and injected with an equal volume of serum-free medium without the aforementioned stimulating peptide pool.
(2) Evaluation criteria: When the spot count of the control wells is ≤ 5 SFC, the spot count of the sample wells shall be ≥ 10 SFC; when the spot count of the control wells ranges from 5 SFC to 10 SFC, the ratio of the spot count of the sample wells to that of the control wells shall be ≥ 2; when the spot count of the control wells is > 10 SFC, the ratio of the spot count of the sample wells to that of the control wells shall be ≥ 3.

5. Experimental Results

**Table 8. Positive conversion rate (%) of gE-specific IFN-γ secreted by splenocytes**

| Group | Vaccine components per mouse | wk02 | wk04 | wk12 | wk24 |
|---|---|---|---|---|---|
| A | PBS | 0 (0/5) | 0 (0/5) | 0 (0/5) | 0 (0/5) |
| B | 1 µg gE antigen | 0 (0/5) | 20 (0/5) | 40 (2/5) | 60 (3/5) |
| C | 1 µg gE antigen + 1 µg QS21 liposome | 80 (4/5) | 20 (1/5) | 40 (2/5) | 60 (3/5) |
| D | 1 µg gE antigen + 10 µg CpG | 80 (4/5) | 100 (5/5) | 40 (2/5) | 80 (4/5) |
| E | 1 µg gE antigen + CpG-QS21 liposomal nanocomposite formulation (CpG: 10 µg, QS21 liposome: 1 µg) | 100 (5/5) | 100 (5/5) | 100 (5/5) | 100 (5/5) |
| F | Commercially available vaccine Shingrix (1 µg gE + 1 µg QS21 liposome + 1 µg MPL) | 80 (4/5) | 100 (5/5) | 100 (5/5) | 100 (5/5) |

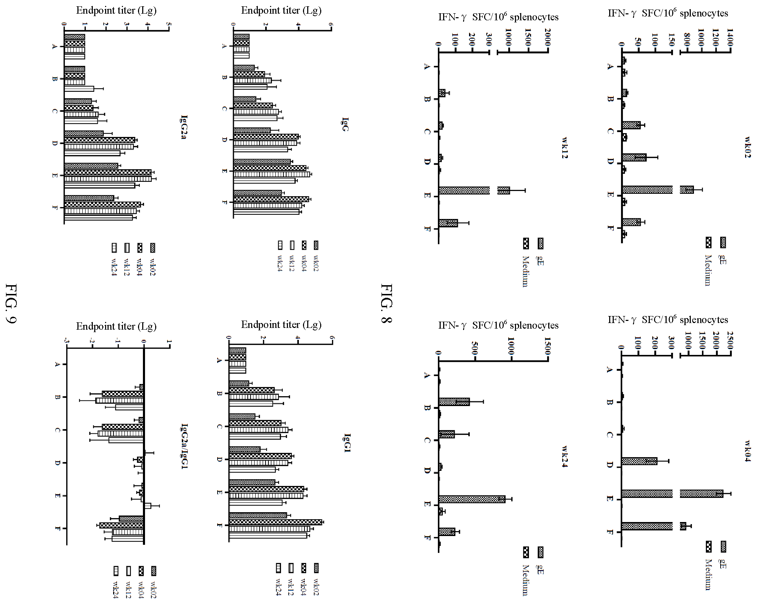

Result analysis: The spot counts of gE-specific IFN-γ-secreting T lymphocytes from mouse splenocytes in each immunized group are shown in FIG. 8, and the positive conversion rates of gE-specific IFN-γ are shown in Table 8. At week 2 (wk02, two weeks after the primary immunization), Group E induced a relatively high level of gE-specific IFN-γ cellular immune response, with a spot count of approximately 900 SFC per 10⁶ splenocytes. After the secondary immunization, the gE-specific IFN-γ cellular immune response level produced was further increased, with the spot count reaching up to 2200 SFC per 10⁶ splenocytes, which was significantly higher than that of the antigen-only group, any single adjuvant group, and the commercially available vaccine Shingrix group. Although the induced gE-specific IFN-γ level decreased over time, a relatively high gE-specific IFN-γ level (approximately 900 SFC per 10⁶ splenocytes) and a 100% positive conversion rate were still maintained at week 24 (wk24).

The results indicate that the QS21 liposome adjuvant and CpG adjuvant contained in the CpG-QS21 liposomal nanocomposite formulation for injection exhibit a favorable synergistic effect in promoting cellular immunity. Moreover, compared with the commercially available vaccine Shingrix, the TVAX-006 vaccine can induce a stronger cellular immune response level.

### 6. Determination of gE-Specific Antibodies against Herpes Zoster in Serum

(1) Detection method: ELISA.
(2) Experimental results: The titer levels of gE-specific IgG/IgG1/IgG2a antibodies in the serum from each immunized group after immunization are shown in FIG. 9. All groups induced detectable gE antigen-specific IgG/IgG1/IgG2a antibody titers at week 2 (wk02, two weeks after the primary immunization). The gE antigen-specific IgG/IgG1/IgG2a antibody titers induced in Group E and Group F were higher than those in any single adjuvant group and the antigen-only group. After the secondary immunization, the gE-specific IgG/IgG1/IgG2a antibody titer levels induced in each group were further elevated. The gE-specific IgG/IgG1/IgG2a antibody titers in Group E and Group F remained at relatively high levels from weeks 4 to 12 (wk04-wk12). The antibody titers in Group E decreased slightly at week 24 (wk24), but were still maintained at relatively high levels (IgG, IgG1 and IgG2a antibody titers were 3.798 Lg, 3.110 Lg and 3.395 Lg, respectively). Group F maintained at relatively high antibody titer levels at week 24 (wk24) (IgG, IgG1 and IgG2a antibody titers were 4.043 Lg, 4.537 Lg and 3.277 Lg, respectively). The IgG2a/IgG1 ratio indicated that the immune responses induced by Group E and the gE antigen combined with CpG adjuvant group were more biased toward the Th1 pathway, whereas those induced by the commercially available vaccine Shingrix group, the antigen-only group, and the gE antigen combined with QS21 liposome adjuvant group were more biased toward the Th2 pathway.

### Example 7: Experimental Group Setting and Immunization Procedure for Hepatitis B Vaccines

1. Experimental animals: Female 6-week-old C57BL/6 mice, n = 48, purchased from Shanghai Lingchang Biotechnology Co., Ltd., with the animal license No. SCXK(Hu) 2018-0003.
2. Animal grouping: The detailed grouping is shown in Table 9. The injection volume per dose was 100 µL per mouse. Before injection, the components listed in the table below for each group were dissolved in PBS solution and diluted to 100 µL. Group A served as the negative control and was injected with 100 µL of PBS per mouse.

**Table 9. Grouping and administration doses**

| Group | Number (mice) | Component (µg per mouse) | | | | | | Administration route |
|---|---|---|---|---|---|---|---|---|
| | | HBsAg | HBcAg | CpG | QS21 | Blank liposome | CpG-QS21 liposomal nanocomposite formulation | |
| A | 8 | | | | | | | Intramuscular injection |
| B | 8 | 20 | 10 | 10 | | | | Intramuscular injection |
| C | 8 | 20 | 10 | | | | 1 & 10* | Intramuscular injection |
| D | 8 | 20 | 10 | | | | | Intramuscular injection |
| E | 8 | 20 | 10 | | | 1 | | Intramuscular injection |
| F | 8 | 20 | 10 | 10 | 1 | | | Intramuscular injection |

| | | | | | | | | |
|---|---|---|---|---|---|---|---|---|
| * In the CpG-QS21 liposome, CpG is 10 µg, and the QS21 liposome is 1 µg. | | | | | | | | |

3. Animal Immunization

C57BL/6 mice were injected with rAAV8-1.3HBV ayw via the tail vein to establish a C57BL/6 mouse model of persistent rAAV8-HBV infection. This HBV infection model was used to evaluate different antiviral drugs, the TVAX-008 injection (HBsAg + HBcAg + CpG, i.e., Group B), the TVAX-028 injection (HBsAg + HBcAg + CpG-QS21 liposome, i.e., Group C), the antigen group (HBsAg + HBcAg, i.e., Group D), the antigen-liposome group (HBsAg + HBcAg + blank liposome, i.e., Group E), and the antigen with free adjuvant group (HBsAg + HBcAg + QS21 + CpG). The PBS group was set as the negative control simultaneously.

Both TVAX-008 injection (HBsAg + HBcAg + CpG) and TVAX-028 injection (HBsAg + HBcAg + CpG-QS21 liposome) were administered intramuscularly once every two weeks.

Dosing frequency and duration: All groups were immunized via intramuscular injection into the posterior thigh at week 6, 8, 10, 12, 14, and 16 (wk06, wk08, wk10, wk12, wk14, wk16), for a total of 6 immunizations.

### 4. Determination of Serum HBsAg Levels for Hepatitis B Vaccines

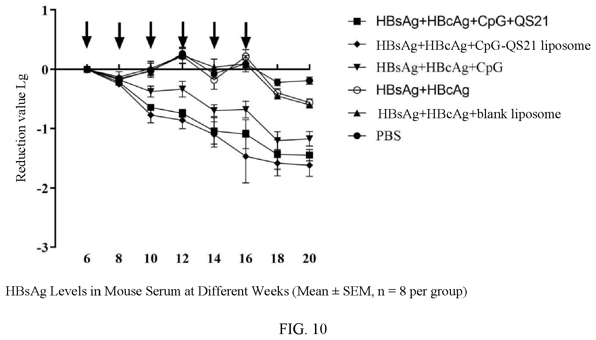

(1) Detection procedure: Refer to the instructions of the BD^{™} ELISPOT Mouse IFN-γ ELISPOT Set.
(2) Detection results: The levels of HBsAg in mouse serum of each group at different determination time points are shown in FIG. 10. The results indicated that, compared with Group A (PBS control group), Group D (antigen group) and Group E (antigen-liposome group), Group B (HBsAg + HBcAg + CpG), Group F (HBsAg + HBcAg + QS21 + CpG) and Group C (HBsAg + HBcAg + CpG-QS21 liposome) effectively reduced the HBsAg levels in HBV infection model mice. Moreover, the declining trend in Group C (HBsAg + HBcAg + CpG-QS21 liposome) was greater than that in Group B (HBsAg + HBcAg + CpG) and Group F (HBsAg + HBcAg + QS21 + CpG).

### 5. Evaluation of Cellular Immune Effect of Hepatitis B Vaccines

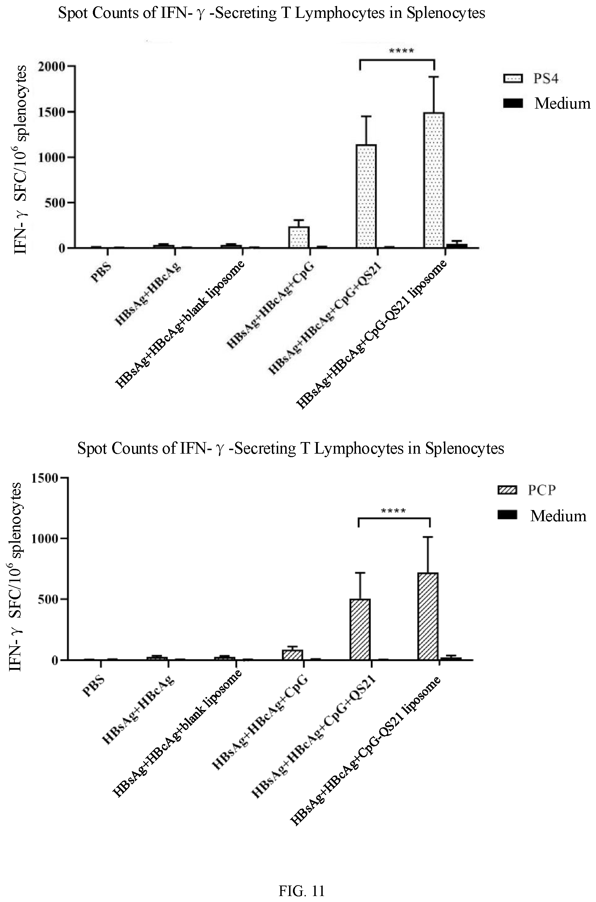

(1) Detection method: T lymphocyte spot counting assay was adopted. Before spot counting, Groups A to C were stimulated with the peptide pools during splenocyte activation. The PS4 and PCP peptide pools, diluted and dissolved in serum-free medium, were used for stimulation and determination, respectively. Meanwhile, a medium negative control group (medium group) was set for each group. For the medium group, splenocytes were isolated from unimmunized mice and injected with an equal volume of serum-free medium.
(2) Detection results: The spot counts of HBsAg-specific and HBcAg-specific IFN-γ-secreting T cells from mouse splenocytes in each group at the experimental endpoint are shown in FIG. 11. The results indicated that compared with the PBS control group, the antigen group and the antigen-liposome group, relatively high levels of HBsAg-specific and HBcAg-specific IFN-γ were determined in Group B (HBsAg + HBcAg + CpG), Group F (HBsAg + HBcAg + QS21 + CpG), and Group C (HBsAg + HBcAg +CpG-QS21 liposome), following stimulation using the PS4 and PCP peptide pools. Moreover, the IFN-γ secretion level in Group C (HBsAg + HBcAg + CpG-QS21 liposome) was significantly higher than that in Group B (HBsAg + HBcAg + CpG) and Group F (HBsAg + HBcAg + QS21 + CpG).

The above are merely several exemplary embodiments of the present invention, and are not intended to limit the present invention in any form. Although the present invention has been disclosed above through the preferred examples, it is not intended to limit thereto. Any equivalent or similar examples obtained by persons skilled familiar with the pertinent art through making minor modifications or alterations using the technical contents disclosed above, without departing from the scope of the technical solutions of the present invention, shall fall within the scope of the present invention.

## Claims

1. An immunological adjuvant composition, comprising an immune activator and a liposome for loading the immune activator, wherein the immune activator comprises a saponin and a CpG oligodeoxynucleotide.

2. The immunological adjuvant composition according to claim 1, wherein the liposome comprises a phospholipid and a sterol;
preferably, the phospholipid is one or more selected from the group consisting of dioleoyl phosphatidylcholine, egg yolk phosphatidylcholine, phosphocholine, or natural phospholipid derivatives; more preferably, the phospholipid is dioleoyl phosphatidylcholine;
preferably, the sterol is one or more selected from the group consisting of cholesterol, stigmasterol, or ergosterol; more preferably, the sterol is cholesterol.

3. The immunological adjuvant composition according to claim 1 or 2, wherein the CpG oligodeoxynucleotide has two or more copies of the 5'-TTCGTT-3' motif or the 5'-TCGTCGTCG-3' motif;
preferably, the CpG oligodeoxynucleotide comprises or consists of a sequence selected from the following:
(1) a nucleotide sequence as set forth in any one of SEQ ID NO: 1 to SEQ ID NO: 13;
(2) a nucleotide sequence having at least 90%, 91%, 92%, 93%, 94%, 95%, 96%, 97%, 98%, 99% or more identity to the nucleotide sequence as set forth in any one of SEQ ID NO: 1 to SEQ ID NO: 13;
(3) a nucleotide sequence having one or more, such as 2, 3, 4 or 5, nucleotide substitutions, deletions or insertions relative to the nucleotide sequence as set forth in any one of SEQ ID NO: 1 to SEQ ID NO: 13;
more preferably, the CpG oligodeoxynucleotide comprises or consists of the nucleotide sequence as set forth in SEQ ID NO: 1, SEQ ID NO: 2 or SEQ ID NO: 3;
more preferably, the CpG oligodeoxynucleotide comprises or consists of the nucleotide sequence as set forth in SEQ ID NO: 1.

4. The immunological adjuvant composition according to any one of claims 1 to 3, wherein the saponin is one or more selected from the group consisting of quillaja saponin, ginsenoside, platycodin, astragaloside, notoginsenoside, glycyrrhizin, albiziae saponin, ophiopogonin, saikosaponin, or panax japonicus saponin;
preferably, the quillaja saponin is one or more selected from the group consisting of QS-7, QS-17, QS-18, or QS-21; more preferably, the quillaja saponin is QS-21.

5. The immunological adjuvant composition according to any one of claims 1 to 4, wherein the weight ratio of the phospholipid to the sterol is (20-4000): (50-1000), preferably (20-2000): (50-500);
preferably, the weight ratio of the combination of the liposome and the saponin to the CpG oligodeoxynucleotide is (0.1-5): (0.5-120), preferably 1: (5-20), and more preferably 1: 10;
preferably, the weight ratio of the saponin to the liposome is (0.1-5): (10-150), preferably (0.2-5): (15-150).

6. The immunological adjuvant composition according to any one of claims 1 to 5, wherein the immunological adjuvant composition is in the form of nanoparticles; preferably, the particle size of the nanoparticles is 20-120 nm.

7. The immunological adjuvant composition according to any one of claims 1 to 6, wherein the immunological adjuvant composition further comprises an antigen, a fragment thereof, a variant thereof, or a mixture of at least two thereof;
preferably, the antigen is one or more selected from the group consisting of a hepatitis A, B, C or E virus antigen, a human herpesvirus antigen, a human immunodeficiency virus antigen, a varicella-zoster virus antigen, a human cytomegalovirus antigen, a respiratory syncytial virus antigen, a human papillomavirus antigen, an influenza virus antigen or a *Mycobacterium tuberculosis* antigen; more preferably, the antigen is a hepatitis B virus antigen or a herpesvirus antigen;
preferably, the hepatitis B virus antigen is a hepatitis B virus surface antigen and a hepatitis B virus core antigen.

8. The immunological adjuvant composition according to any one of claims 1 to 7, wherein
the weight ratio of the herpesvirus antigen to the combination of the liposome and the saponin to the CpG oligodeoxynucleotide is (0.1-5): (0.1-5): (0.5-120), preferably (1-2): 1: (5-20), and more preferably 1: 1: 10;
the weight ratio of the hepatitis B virus antigen to the combination of the liposome and the saponin to the CpG oligodeoxynucleotide is (5-100): (0.1-5): (0.5-120), preferably (15-50): 1: (5-20), and more preferably 30: 1: 10.

9. A method for preparing the immunological adjuvant composition according to any one of claims 1 to 8, comprising the following steps:
dissolving the saponin in an aqueous solution, adding the liposome thereto, performing a first stirring, then adding the CpG oligodeoxynucleotide and performing a second stirring to obtain the immunological adjuvant composition.

10. The method for preparing the immunological adjuvant composition according to claim 9, wherein the stirring speed of each of the first stirring and the second stirring is independently 50-150 rpm, preferably 50-100 rpm;
preferably, the aqueous solution comprises a phosphate and sodium chloride;
preferably, the concentration of phosphate in the aqueous solution is 10-50 mmol/L, preferably 20 mmol/L; the concentration of sodium chloride in the aqueous solution is 100-350 mmol/L, preferably 150 mmol/L.

11. The method for preparing the immunological adjuvant composition according to claim 9 or 10, wherein the method further comprises a step of adding an antigen after the second stirring.

12. An immunological kit, comprising the immunological adjuvant composition according to any one of claims 1 to 8.

13. Use of the immunological adjuvant composition according to any one of claims 1 to 8 or the immunological kit according to claim 12 in the manufacture of the following products:
(1) a medicament for the prevention and/or treatment of a human herpesvirus and/or hepatitis B virus infection or a related disease thereof; preferably, the medicament is a vaccine;
(2) a kit for diagnosing a human herpesvirus and/or hepatitis B virus infection; or
(3) an immunogen for developing an antibody against a human herpesvirus and/or a hepatitis B virus;
preferably, the human herpesvirus is one or more selected from the group consisting of a varicella-zoster virus, a herpes simplex virus type 1, and a herpes simplex virus type 2.
